# EUROPEAN PATENT APPLICATION

(11) **EP 0 664 301 A1**
(43) Date of publication of application: **26.07.1995**
(21) Application number: 94100976.3
(22) Date of filing: 24.01.1994
(51) Int. Cl.: C08B 37/08, A61K 7/06

(54) **Chitosan derivatives, preparation process thereof and cosmetic compositions containing same**

(71) Applicant: DAINICHISEIKA COLOR & CHEMICALS MFG. CO. LTD., Chuo-ku Tokyo 103 (JP)
(72) Inventor: Tsuchida, Shinya, Saitama-ken (JP); Ise, Hiroshi, Cyofu-shi, Tokyo (JP); Sannan, Takanori, Suginami-ku, Tokyo (JP); Horiguchi, Shojiro, Omiya-shi, Saitama-ken (JP)
(74) Representative: Wächtershäuser, Günter, Prof. Dr.

(57) **Abstract**

Described are chitosan derivatives which are each obtained by reacting at least one alkylene oxide such as ethylene oxide, propylene oxide or butylene oxide or a mixture thereof with chitosan having a deacylation degree of at least 90%, preferably 95-100% and a molecular weight not greater than 400,000, preferably of from 10,000 to 100,000. Cosmetic compositions containing such chitosan derivatives are also described.

## Description

### BACKGROUND OF THE INVENTION

### a) Field of the Invention

This invention relates to chitosan derivatives and a preparation process thereof, and more specifically to chitosan derivatives excellent in long-term storage clarity when formed into solutions and hence useful as raw materials for cosmetic compositions. This invention is also concerned with a preparation process of such chitosan derivatives and cosmetic compositions containing the same.

### b) Description of the Related Art

Hydroxypropylated chitosan, one of hydroxypropylated deacetylated chitins, has been disclosed, for example, in Japanese Patent Kokai No. SHO 64-005601, in which the hydroxypropylated chitosan is described useful as a water-soluble high-molecular substance in various applications.

Chitosan, the starting raw material of the hydroxypropylated chitosan, is however of such a grade that its deacetylation degree generally ranges from 70% to 80% or so, thereby involving the problems that the hydroxypropylation reaction itself requires setting of irksome conditions such as use of an organic solvent and pH control and the resulting hydroxypropylated chitosan is not uniform but contains many impurities.

Further, the hydroxypropylated chitosan so obtained is in a liquid form having good clarity immediately after its preparation but its clarity drops with the time. This has made it undesirous to use the hydroxypropylated chitosan as a raw material for cosmetic compositions or as a food additive. This problem is attributed to the inclusion of many byproducts formed upon hydroxypropylation of chitosan in water. Because these byproducts are also water-soluble like the hydroxypropylated chitosan, it is difficult to remove the byproducts from the hydroxypropylated chitosan by washing them with water.

The above purity problem of hydroxypropylated chitosan may be overcome to a certain extent if the hydroxypropylated chitosan is purified by subjecting it to fractional precipitation many times while using an organic solvent having appropriate solubility or by using a special apparatus such as a dialyzer. However these purification methods are anyhow costly and can therefore hardly be used industrially.

### SUMMARY OF THE INVENTION

An object of the present invention is to provide a chitosan derivative which does not contain much impurities and, even when formed into an aqueous solution or an alcohol solution, can retain high clarity for a long time.

Another object of the present invention is to provide a preparation process for the chitosan derivative.

A further object of the present invention is to provide a cosmetic composition comprising the chitosan derivative.

These objects can be achieved by the present invention.

In one aspect of the present invention, there is thus provided a chitosan derivative obtained by reacting at least one alkylene oxide with chitosan having a deacetylation degree of at least 90% and a molecular weight not greater than 400,000.

In another aspect of the present invention, there is also provided a process for the preparation of a chitosan derivative, which comprises reacting at least one alkylene oxide with chitosan having a deacetylation degree of at least 90% and a molecular weight not greater than 400,000 while using water as a medium.

In a further aspect of the present invention, there is also provided a process for the preparation of a chitosan derivative, which comprises reacting chitosan, which has a deacetylation degree of at least 90% and a molecular weight not greater than 400,000, with at least one alkylene oxide to an intermediate extent of the reaction, once dissolving a partial reaction product, which has been formed, in an aqueous or organic solvent, and then resuming the reaction. If necessary or desired, the reaction can be resumed after the partial reaction product is caused to deposit.

In a still further aspect of the present invention, there is also provided a cosmetic composition comprising a solution of the above chitosan derivative in a cosmetologically acceptable solvent such as an aqueous solvent or an alcohol.

Owing to the use of chitosan having a deacetylation degree of at least 90% and a molecular weight not greater than 400,000 as the raw material chitosan, the reaction with the alkylene oxide can be conducted in water without using an organic solvent and the percentage of addition of the alkylene oxide is high. It is therefore possible to provide a chitosan derivative which, when formed into an aqueous solution or an alcohol solution, can retain clarity for a long period without developing turbidity.

### DETAILED DESCRIPTION OF THE INVENTION AND PREFERRED EMBODIMENTS

Chitosan useful in the present invention, which has a deacetylation degree of at least 90% and a molecular weight not greater than 400,000, is a deacetylated product of chitin - a sort of natural high-molecular substance found in marine invertebrates such as crabs, lobsters and shrimps, arthropods such as insects, fungi and the like - and is a basic polysaccharide containing 2-amino-2-deoxy-D-glucose as a recurring structural unit.

Such deacetylated chitin itself has already been produced industrially and is available in various grades on the market. The deacetylation degrees of industrially available chitosan products are in a range of from about 70% to about 80% or so. Chitosan usable for the objects of the present invention is however one having a deacetylation degree of at least 90%.

To obtain chitosan having a deacetylation degree of at least 90%, chitin is subjected to deacetylation with a concentrated alkali solution and, after removal of the alkali solution, the deacetylation product is washed with water and is then treated with a concentrated alkali solution, as is described in detail in Japanese Patent Publication (Kokoku) No. SHO 56-34201 or 58-00441. These operations are repeated until a desired deacetylation degree is achieved. Particularly preferred chitosan is substantially-deacetylated chitosan whose deacetylation degree ranges from 95% to 100%.

Illustrative alkylene oxide usable in the present invention include ethylene oxide, propylene oxide and butylene oxide, with propylene oxide being particularly preferred. Preferably these alkylene oxides are used in a proportion of about 5-20 moles per amino group of chitosan. If the alkylene oxide is used too little, the resulting product has insufficient solubility in water. No extra advantage can however be brought about even if the alkylene oxide is employed in any unduly large proportion.

Although the above hydroxyalkylation of chitosan may be conducted in an aqueous medium containing an alcoholic organic solvent such as isopropyl alcohol, it can be conducted readily in water free of any organic solvent. As the most preferably reaction method, the above chitosan is suspended in water and then reacted at 50-100°C for 3-24 hours or so. The dispersed chitosan raw material becomes swollen as the hydroxyalkylation proceeds. It is however necessary to proceed with the reaction as is, whereby the target product can be obtained.

In a preferred embodiment, a suitable mineral acid or organic acid, for example, an inorganic acid such as hydrochloric acid, sulfuric acid, phosphoric acid or sulfamic acid, an organic acid such as acetic acid, lactic acid, citric acid, adipic acid or pyrrolidonecarboxylic acid, or a mixture thereof is added to a reaction system, in which the chitosan is contained in a swollen and dispersed state, in the course of the reaction to adjust the pH to 2-5, so that the reaction product is dissolved completely in water. The reaction is then resumed either without changing the pH of the reaction system or after adjusting it back from a slightly acidic state to an alkaline state, thereby making it possible to highly hydroxyalkylate the chitosan.

The chitosan derivative according to the present invention obtained as described above has a molecular weight preferably not higher than 400,000. A more preferred range is from 10,000 to 100,000. Control of the molecular weight to the above range can be effected by heating and treating the chitosan raw material or the product in an aqueous solution of an oxidizing agent such as sodium perborate.

A molecular weight higher than 400,000, when formed into an aqueous solution of an alcohol solution, leads to a higher viscosity, thereby making it difficult to obtain a high-density solution of a chitosan derivative. It is not easy to use chitosan derivatives of such a high molecular weight as raw materials for cosmetic compositions.

The cosmetic composition according to the present invention can be formulated by dissolving such a chitosan derivative of the present invention as described above in water, a mixed solvent of water and an organic solvent such as an alcohol, or an alcohol and adding and mixing a perfume, a colorant and other auxiliary ingredients as needed. The preferred concentration of the chitosan derivative is 0.1-0.5 wt.%. The cosmetic composition according to the present invention is suited for the treatment of human hair and, when applied by spraying or the like, can impart excellent moisturized and smooth touch feeling and good combing smoothness to the hair although the hair so treated looks non-greasy and rather dry.

The present invention will hereinafter be described more specifically by the following Examples and Comparative Examples, in which all the designations of "parts" or "part" mean parts or part by weight unless otherwise specifically indicated. The long-term storage clarity of the solution of the product in each of the Examples and Comparative Examples was determined in accordance with the following testing conditions and ranking standard:

The product of each Example or Comparative Example was dissolved at a concentration of 5 wt.% in 95% ethanol. 100 mℓ of the resulting solution were placed in a glass bottle having a capacity of 150 mℓ. After the bottle was left over at 25°C for 3 months at a dark place, the solution was taken out and observed by the naked eye. The observation results were ranked in accordance with the following standard:
A: the clarity was excellent,
B: the clarity was good, and
C: the solution was opaque or turbid.

### Example 1

An autoclave was charged with 50 parts of chitosan (deacetylation degree: 95%, average molecular weight: 40,000), 300 parts of water, 6 parts of a 20% aqueous solution of sodium hydroxide and 300 parts of propylene oxide. They were allowed to react at 70°C for 20 hours under stirring. The reaction mixture was cooled and then adjusted to pH 8-9. The reaction mixture was added dropwise under stirring into 5 liters of hot water of 90°C, whereby the target product, hydroxypropylchitosan, was caused to precipitate. The target product was washed with 5 liters of hot water, dried at 50°C and then ground, so that 81 parts of hydroxypropylchitosan powder were obtained. Further, to determine the long-term storage clarity of the resultant hydroxypropylchitosan powder, it was dissolved at a concentration of 5% in 95% ethanol. The long-term storage clarity of the solution so formed was then checked. The solution was found to retain excellent clarity for a long time.

### Example 2

An autoclave was charged with 50 parts of chitosan (deacetylation degree: 95%. average molecular weight: 40,000), 150 parts of water and 100 parts of propylene oxide. The contents were gradually heated to 100°C under stirring. When they were allowed to react for 8 hours under the same conditions, the pressure dropped from 5 kgG/cm² at the beginning to 0.5 kgG/cm² at the end of the reaction.

The reaction mixture was once cooled, to which 20 parts of pyrrolidonecarboxylic acid (PCA) were added at room temperature. The resulting mixture was stirred for 4 hours so that the reaction mixture was dissolved. Thirty-seven parts of a 20% aqueous solution of sodium hydroxide were added dropwise to neutralize the reaction mixture. Two hundred parts of propylene oxide were added, followed by a reaction at 70°C for 8 hours. The reaction mixture so obtained was cooled, and its pH was then adjusted to 8-9. The reaction mixture was added dropwise under stirring into 5 liters of hot water of 90°C, whereby the target product, hydroxypropylchitosan, was caused to precipitate. The target product was washed with 5 liters of hot water, dried at 50°C and then ground, so that 80 parts of hydroxypropylchitosan powder were obtained.

To determine the long-term storage clarity of a solution of the resultant hydroxypropylchitosan powder, it was dissolved at a concentration of 5% in 95% ethanol. The long-term storage clarity of the solution so formed was then checked. The solution was found to retain excellent clarity for a long time.

### Examples 3-6

In each Example, hydroxyalkylchitosan powder was obtained in a similar manner to Example 1 or 2 by changing the reaction conditions in various ways as shown in Table 1 and Table 2. An ethanol solution of hydroxyalkylchitosan powder obtained in Example 3 had good long-term storage clarity, while ethanol solutions of hydroxyalkylchitosan powders obtained in Examples 4-6, respectively, had excellent long-term storage clarity.

### Comparative Examples 1-3

In each Comparative Example, hydroxyalkylchitosan powder was obtained in a similar manner to Example 1 or 2 by changing the reaction conditions in various ways as shown in Table 3.

Comparative Examples 1 and 2 were conducted in the same manner as in Examples 1 and 2 except for the replacement of the raw material chitosan by 75%- and 80%-deacetylated chitosan products, respectively. The yields were low, and solutions of the resultant hydroxyalkylchotosans had insufficient clarity.

Comparative Example 3 was conducted in the same manner as in Example 1 except for the use of chitosan having a molecular weight of 500,000 as a raw material. As the reaction proceeded, the reaction mixture became highly viscous so that it was difficult to stir the reaction mixture. When purified by hot water, the yield was extremely low due to inclusion of soluble components. Even in a solution of the hydroxyalkylchitosan, some gel was contained and the clarity was poor.

**Table 1**

| | Example 1 | Example 2 | Example 3 |
|---|---|---|---|
| Chitosan | 50 parts | 50 parts | 50 parts |
| Deacetylation degree | 95% | 95% | 95% |
| Average molecular weight | 100,000 | 40,000 | 40,000 |
| Water | 300 parts | 150 parts | 150 parts |
| Propylene oxide | - | 100 parts | 100 parts |
| Butylene oxide | - | - | - |
| Reaction (1) | - | 100°C/8 hrs | 70°C/20 hrs |
| PCA | - | 20 parts | - |
| Acetic acid | - | - | - |
| 20% NaOH (per mole of acid) | 6 parts | 37 parts | 6 parts |
| | - | 1.2 moles | - |
| Propylene oxide | 300 parts | 200 parts | 200 parts |
| Butylene oxide | - | - | - |
| Reaction (2) | 70°C/20 hrs | 70°C/8 hrs | 70°C/8 hrs |
| Yield | 81 parts | 80 parts | 82 parts |
| Long-term storage clarity of solution | B | A | B |

**Table 2**

| | Example 4 | Example 5 | Example 6 |
|---|---|---|---|
| Chitosan | 50 parts | 50 parts | 50 parts |
| Deacetylation degree | 90% | 98% | 95% |
| Average molecular weight | 100,000 | 200,000 | 40,000 |
| Water | 300 parts | 400 parts | 150 parts |
| Propylene oxide | 100 parts | - | 50 parts |
| Butylene oxide | - | 100 parts | 50 parts |
| Reaction (1) | 100°C/8 hrs | 70°C/20 hrs | 70°C/20 hrs |
| PCA | - | 20 parts | 20 parts |
| Acetic acid | 10 parts | - | - |
| 20% NaOH (per mole of acid) | 31.3 parts | 29.5 parts | 37 parts |
| | 1.0 mole | 0.95 mole | 1.2 moles |
| Propylene oxide | - | - | 100 parts |
| Butylene oxide | 150 parts | 200 parts | 100 parts |
| Reaction (2) | 70°C/15 hrs | 70°C/15 hrs | 70°C/8 hrs |
| Yield | 78 parts | 86 parts | 88 parts |
| Long-term storage clarity of solution | A | A | A |

**Table 3**

| | Comparative Example 1 | Comparative Example 2 | Comparative Example 3 |
|---|---|---|---|
| Chitosan | 50 parts | 50 parts | 50 parts |
| Deacetylation degree | 80% | 75% | 95% |
| Average molecular weight | 100,000 | 50,000 | 500,000 |
| Water | 300 parts | 150 parts | 300 parts |
| Propylene oxide | - | 100 parts | - |
| Butylene oxide | - | - | - |
| Reaction (1) | | 70°C/20 hrs | - |
| PCA | - | - | - |
| Acetic acid | - | - | - |
| 20% NaOH (per mole of acid) | 6 parts | 6 parts | 6 parts |
| | - | - | - |
| Propylene oxide | 300 parts | 200 parts | 300 parts |
| Butylene oxide | - | - | - |
| Reaction (2) | 70°C/20 hrs | 70°C/8 hrs | 70°C/20 hrs |
| Yield | 74 parts | 76 parts | 55 parts |
| Long-term storage clarity of solution | C | C | C |

### Example 7

An autoclave was charged with 50 parts of chitosan (deacetylation degree: 95%. average molecular weight: 40,000), 150 parts of water and 100 parts of propylene oxide. The contents were gradually heated to 100°C under stirring. When they were allowed to react for 8 hours under the same conditions, the pressure dropped from 5 kgG/cm² at the beginning to 0.5 kgG/cm² at the end of the reaction.

The reaction mixture was once cooled, to which 20 parts of pyrrolidonecarboxylic acid (PCA) were added at room temperature. The resulting mixture was stirred for 4 hours so that the reaction mixture was dissolved. Thirty-seven parts of a 20% aqueous solution of sodium hydroxide were added dropwise to neutralize the reaction mixture. Two hundred parts of propylene oxide were added, followed by a reaction at 70°C for 8 hours. The reaction mixture so obtained was cooled, and its pH was then adjusted to 8-9. The reaction mixture was added dropwise under stirring into 5 liters of hot water of 90°C, whereby the target product, hydroxypropylchitosan, was caused to precipitate. The target product was washed with 5 liters of hot water, dried at 50°C and then ground, so that 80 parts of hydroxypropylchitosan powder were obtained.

To determine the long-term storage clarity of a solution of the resultant hydroxypropylchitosan powder, it was dissolved at a concentration of 5% in 95% ethanol. The long-term storage clarity of the solution so formed was then checked. The solution was found to retain good clarity for a long time.

### Examples 8-11

In each Example, hydroxyalkylchitosan powder was obtained in a similar manner to Example 7 by changing the reaction conditions in various ways as shown in Table 4 and Table 5. An ethanol solution of hydroxyalkylchitosan powder obtained in each Example had excellent long-term storage clarity.

### Comparative Examples 4-6

In each Comparative Example, hydroxyalkylchitosan powder was obtained in a similar manner to Example 7 by changing the reaction conditions in various ways as shown in Table 4 and Table 5.

Comparative Example 4 was conducted in a similar manner to Example 7 except that the raw material chitosan was replaced by an 80%-deacetylated chitosan product and the reaction was conducted in one step without carrying out dissolution with an acid in the course of the reaction.

Comparative Example 5 was conducted in a similar manner to Example 7 except that the raw material chitosan was replaced by a 75%-deacetylated chitosan product and the reaction was conducted in two steps without carrying out dissolution with an acid in the course of the reaction.

In both Comparative Examples 4 and 5, the yields were poor and solutions of the resultant hydroxyalkylchitosans were opaque.

Comparative Example 6 was conducted in a similar manner to Example 7 except for the replacement of the raw material chitosan by one having a molecular weight of 500,000. As the reaction proceeded, the reaction mixture became highly viscous so that it was difficult to stir the reaction mixture. When purified by hot water, the yield was extremely low due to inclusion of soluble components. Even in a solution of the hydroxyalkylchitosan, some gel was contained and the solution was turbid.

**Table 4**

| | Example 7 | Example 8 |
|---|---|---|
| Chitosan | 50 parts | 50 parts |
| Deacetylation degree | 95% | 95% |
| Average molecular weight | 40,000 | 100,000 |
| Water | 150 parts | 75 parts |
| Isopropyl alcohol | - | 75 parts |
| Propylene oxide | 100 parts | - |
| Butylene oxide | - | 100 parts |
| Reaction (1) | 100°C/8 hrs | 70°C/20 hrs |
| PCA | 20 parts | 20 parts |
| Acetic acid | - | - |
| 20% NaOH (per mole of acid) | 37 parts | 30.4 parts |
| | 1.2 moles | 0.98 mole |
| Propylene oxide | 200 parts | - |
| Butylene oxide | - | 200 parts |
| Reaction (2) | 70°C/8 hrs | 70°C/15 hrs |
| Yield | 80 parts | 86 parts |
| Long-term storage clarity of solution | B | A |

**Table 5**

| | Example 9 | Example 10 | Example 11 |
|---|---|---|---|
| Chitosan | 50 parts | 50 parts | 50 parts |
| Deacetylation degree | 95% | 98% | 95% |
| Average molecular weight | 100,000 | 200,000 | 40,000 |
| Water | 300 parts | 400 parts | 150 parts |
| Propylene oxide | 100 parts | - | 50 parts |
| Butylene oxide | - | 100 parts | 50 parts |
| Reaction (1) | 100°C/8 hrs | 70°C/20 hrs | 70°C/20 hrs |
| PCA | - | 20 parts | 20 parts |
| Acetic acid | 10 parts | - | - |
| 20% NaOH (per mole of acid) | 31.3 parts | 29.5 parts | 37 parts |
| | 1.0 mole | 0.95 mole | 1.2 moles- |
| Propylene oxide | - | - | 100 parts |
| Butylene oxide | 150 parts | 200 parts | 100 parts |
| Reaction (2) | 70°C/15 hrs | 70°C/15 hrs | 70°C/8 hrs |
| Yield | 78 parts | 86 parts | 88 parts |
| Long-term storage clarity of solution | A | A | A |

**Table 6**

| | Comparative Example 4 | Comparative Example 5 | Comparative Example 6 |
|---|---|---|---|
| Chitosan | 50 parts | 50 parts | 50 parts |
| Deacetylation degree | 80% | 75% | 95% |
| Average molecular weight | 40,000 | 40,000 | 500,000 |
| Water | 150 parts | 150 parts | 300 parts |
| Propylene oxide | - | 100 parts | 100 parts |
| Butylene oxide | - | - | - |
| Reaction (1) | | 70°C/20 hrs | 100°C/8 hrs |
| PCA | - | - | 20 |
| Acetic acid | - | - | - |
| 20% NaOH (per mole of acid) | 6 parts | 6 parts | 37 parts |
| | - | - | 1.2 moles |
| Propylene oxide | 300 parts | 200 parts | 200 parts |
| Butylene oxide | - | - | - |
| Reaction (2) | 70°C/20 hrs | 70°C/8 hrs | 70°C/8 hrs |
| Yield | 74 parts | 76 parts | 50 parts |
| Long-term storage clarity of solution | C | C | C |

### Example 12 (Application to Hair Spray)

### Solution A

| | |
|---|---|
| Hydroxypropylchitosan obtained in Example 1 | 5 parts |
| 95% Ethanol | 95 parts |

In accordance with the following compositions, solution A, dimethyl ether and butane were filled in a spray can.

| | |
|---|---|
| Solution A | 25 parts |
| Dimethyl ether | 35 parts |
| Butane | 40 parts |

When applied to hair, moisturized feeling, high gloss and good set retention were obtained.

### Example 13 (Application to Clear Shampoo)

| | |
|---|---|
| Cocamidopropylbetaine (35%) | 40 parts |
| Water | 54 parts |
| Hydroxypropylhydroxybutylchitosan obtained in Example 2 | 2 parts |
| "Glucamate DOE-120" (trade name; surfactant; product of Union Carbide Corporation) | 3 parts |
| Methylparaben | 1 part |

When hair was washed with a shampoo of the above composition, good combing smoothness and excellent conditioning effects were exhibited.

### Example 14 (Application to Hair Spray)

### Solution A

| | |
|---|---|
| Hydroxypropylchitosan obtained in Example 7 | 5 parts |
| 95% Ethanol | 95 parts |

In accordance with the following composition, solution A, dimethyl ether and butane were filled in a spray can.

| | |
|---|---|
| Solution A | 25 parts |
| Dimethyl ether | 35 parts |
| Butane | 40 parts |

When applied to hair, moisturized feeling, high gloss and good set retention were obtained.

### Example 15 (Application to Clear Shampoo)

| | |
|---|---|
| Cocamidopropylbetaine (35%) | 40 parts |
| Water | 54 parts |
| Hydroxypropylhydroxybutylchitosan obtained in Example 8 | 2 parts |
| "Glucamate DOE-120" | 3 parts |
| Methylparaben | 1 part |

When hair was washed with a shampoo of the above composition, good combing smoothness and excellent conditioning effects were exhibited.

## Claims

1. A chitosan derivative obtained by reacting at least one alkylene oxide with chitosan having a deacetylation degree of at least 90% and a molecular weight not greater than 400,000.

2. A chitosan derivative according to claim 1, wherein the chitosan has a deacetylation degree of from 95% to 100%.

3. A chitosan derivative according to claim 1, wherein the chitosan has a deacetylation degree of from 95% to 100% and a molecular weight of from 10,000 to 100,000.

4. A process for the preparation of a chitosan derivative, which comprises reacting at least one alkylene oxide with chitosan having a deacetylation degree of at least 90% and a molecular weight not greater than 400,000 while using water as a medium.

5. A process according to claim 4, wherein the chitosan has a deacetylation degree of at least 90%.

6. A process according to claim 4, wherein the chitosan has a deacetylation degree of from 95% to 100%.

7. A process according to claim 4, wherein the chitosan has a deacetylation degree of from 95% to 100% and a molecular weight of from 10,000 to 100,000.

8. A process according to claim 4, wherein the acetylene oxide is ethylene oxide, propylene oxide or butylene oxide or a mixture thereof.

9. A process according to claim 4, wherein before the reaction is completed, an acid is added to a reaction system to once dissolve the resultant chitosan derivative and the reaction is then resumed.

10. A process according to claim 9, wherein the acid is hydrochloric acid, sulfuric acid, phosphoric acid, sulfamic acid, acetic acid, lactic acid, citric acid, adipic acid or pyrrolidonecarboxylic acid or a mixture thereof.

11. A process according to claim 9, wherein the reaction is resumed in the presence of an alkali.

12. A process according to claim 11, wherein the acid is hydrochloric acid, sulfuric acid, phosphoric acid, sulfamic acid, acetic acid, lactic acid, citric acid, adipic acid or pyrrolidonecarboxylic acid or a mixture thereof.

13. A process for the preparation of a chitosan derivative, which comprises reacting chitosan, which has a deacetylation degree of at least 90% and a molecular weight not greater than 400,000, with at least one alkylene oxide to an intermediate extent of the reaction, once dissolving a partial reaction product, which has been formed, in an aqueous or organic solvent, and then resuming the reaction.

14. A process according to claim 13, wherein the reaction is resumed after the partial reaction product is caused to deposit.

15. A process according to claim 13, wherein the chitosan has a deacetylation degree of from 95% to 100%.

16. A process according to claim 13, wherein the chitosan has a deacetylation degree of from 95% to 100% and a molecular weight of from 10,000 to 100,000.

17. A process according to claim 13, wherein the acetylene oxide is ethylene oxide, propylene oxide or butylene oxide or a mixture thereof.

18. A cosmetic composition comprising a solution of the chitosan derivative according to claim 1 in a cosmetologically acceptable solvent.

19. A cosmetic composition according to claim 18, wherein the cosmetologically acceptable solvent is an aqueous solvent.

20. A cosmetic composition according to claim 18, wherein the cosmetologically acceptable solvent is an alcohol.
